# EUROPEAN PATENT SPECIFICATION

(11) **EP 0 492 747 B1**
(45) Date of publication and mention of the grant of the patent: **20.03.1996**
(21) Application number: 91203442.8
(22) Date of filing: 26.11.1991
(51) Int. Cl.: A61K 31/455, A61K 9/06

(54) **Pharmaceutical preparation for topical application**
Pharmazeutische Zubereitung zur topischen Anwendung
Préparation pharmaceutique pour application topique

(30) Priority: 26.11.1990 DE 4037554
(43) Date of publication of application: 01.07.1992
(73) Proprietor: ROEMMERS S.A.I.C.F., (1230) Buenos Aires (AR)
(72) Inventor: Vasquez, Carlos Eduardo M., Buenos Aires (AR)
(74) Representative: Behrens, Dieter, Dr.-Ing.

(56) References cited:
- EP-A- 0 105 635
- EP-A- 0 152 379
- FR-A- 2 262 967
- US-A- 4 273 777

## Description

The invention relates to pharmaceutical preparations for topical application which are preferably in the form of creams or gels and contain as active substance the L-lysine salt of the 2-[(3-chloro-2-methylphenyl)amino]-3-pyridine carboxylic acid of the formula (I), hereinafter designated lysine clonixinate.

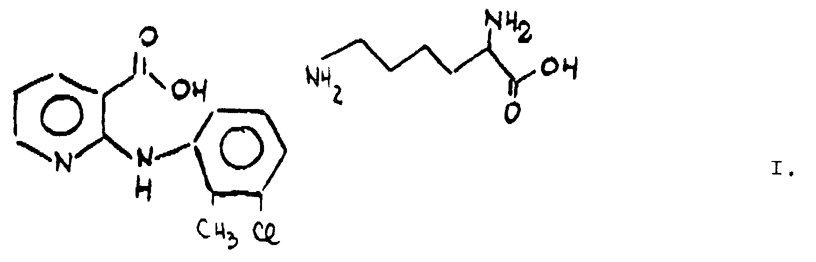

The pharmaceutical preparations are useful as topical analgesics and anti-inflammatory agents.

Drugs for oral and rectal administration and injectable solutions containing lysine clonixinate as active substance are known from the literature, for example from DE-PS 2,253,134 and US-PS 3,973,027 and/or are available on the market. These drugs develop a good analgesic and anti-inflammatory activity. However, there are cases in which they are not suitable or applicable. Thus, when a person suffers from pain in a limited area of the body, for example after a blow or knock or due to a minor accident, in the case of contusions, distortions, luxations or arthritis, arthrosis, tenalgia, bursitis, myalgia and sport injuries, a localized topical application of a substance is generally regarded as the most suitable way or the most suitable method for healing. It is also important not to apply an active substance more strongly or more weakly than is necessary to relieve or eliminate the pain or inflammation, and thus to avoid unnecessary secondary reactions or habituation to the active substances, or dependence thereon, or obtaining only slight relief. Finally, topical formulations such as creams or gels are less dangerous than tablets, in particular for self-medication, as is made possible by freely purchasable remedies. Thus, the making available of remedies for topical application which develop a mean analgesic and anti-inflammatory activity is an important problem in therapeutics.

Topical formulations containing the analgesic/anti-inflammatory agent as active substances are generally known from the literature. For example, NL-OS 8,100,917 (1982), CH-PS 651,474 (1985) and US-PS 4,407,824 (1983) relate to the amine salts of diclofenac. In DE-OS 2,935,776 (1981) as well various organic salts of anti-inflammatory medicaments are named which are suitable for topical application.

It has now been found that the L-lysine salt of 2-[(3-chloro-2-methylphenyl)amino]-3-pyridine carboxylic acid is very well suited to the preparation of pharmaceutical compositions for topical application and develops a very good effect on such an application.

The subject of the invention is therefore pharmaceutical preparations for topical application, preferably in the form of creams or gels, which with respect to the weight contain as active substance 1 to 10 % by weight L-lysine salt of the 2-[(3-chloro-2-methylphenyl)amino]-3-pyridine carboxylic acid and 2 to 15 % by weight pharmaceutically acceptable emulsifiers, 2 to 20 % by weight pharmaceutically acceptable emollients or softening agents, 0.01 to 0.2 % by weight pharmaceutically acceptable preservation substances and water to make up 100 % by weight. Possibly, these agents may also contain 0.2 to 5 % by weight rubefacients and in the case of creams 3 to 18 % by weight pharmaceutically acceptable lubricants or in the case of gels 0.5 to 5 % by weight pH-value modifiers, the accompanying water always being adjusted to make up 100 % by weight.

The pharmaceutical preparations according to the invention are distinguished by the following advantages:
- local action,
- rapid response/reaction due to rapid absorption,
- low toxicity in uninjured or undamaged tissues or organs.

According to an preferred embodiment the pharmaceutical preparations according to the invention for topical application contain 3 to 7 % by weight L-lysine salt of the 2-[(3-chloro-2-methylphenyl)amino]-3-pyridine carboxylic acid as active substance, 3 to 13 % by weight pharmaceutically acceptable emulsifiers, 3 to 10 % by weight pharmaceutically acceptable emollients, 0.02 to 0.1 % by weight pharmaceutically acceptable preservation substances and water as remainder up to 100 %. Possibly, 0.5 to 2 % by weight rubefacients may also be contained therein and in the case of creams 8 to 12 % by weight pharmaceutically acceptable lubricants and in the case of gels 1 to 3 % by weight modifiers of the pH-value, the accompanying water always being made up to a total of 100 %.

Advantageously, the emulsifiers are selected from the group of the following substances: Glyceryl monostearate, glyceryl monostearate/polyoxyethylene stearate, ketostearyl alcohol/potassium lauryl sulfate (emulsifier wax) and carbomer.

The softening agent or emollient is preferably selected from propylene glycol, glycerol, sorbite and isopropyl myristate.

As preservative agents or substance, methyl parabene, ethyl parabene and propyl parabene have proved expedient.

Menthol is advantageously used as rubefacient.

As already mentioned, the pharmaceutical preparations according to the invention are preferably brought into the galenic form of a cream or a gel.

If the pharmaceutical preparation according to the invention is to be formulated as cream, as emulsifier advantageously ketostearyl alcohol/lauryl sulfate ("emulsifier wax") is used and the lubricants are selected from the group consisting of mineral oil, vaseline and fatty acids.

If the pharmaceutical preparations according to the invention are formulated as gel, advantageously carbomer is used as emulsifier and propylene glycol as softening agent, and the modifiers for the pH-value or buffer are selected from the group consisting of sodium hydroxide, triethanol amine, trimethamine, (tris(hydroxymethyl)amino methane) and lysine.

For both creams and gels, isopropyl myristate is very well suited as softening agent or emollient.

Of the preservation agents, methyl parabene and propyl parabene are preferred.

According to the invention, the L-lysine salt of the 2-[(3-chloro-2-methylphenyl)amino]-3-pyridine carboxylic acid can be used for the preparation of a pharmaceutical composition for topical application in order to relieve or at least alleviate pain and/or inflammations in humans and animals.

The following examples serve for more detailed explanation of the invention.

### Example 1: Topical pharmaceutical preparation in the form of a cream without rubefacient.

A cream was made having the following composition:

| | |
|---|---|
| - lysine clonixinate | 5.0 % by weight |
| - isopropyl myristate | 9.0 % by weight |
| - methyl parabene | 0.1 % by weight |
| - emulsifier wax | 13.0 % by weight |
| - mineral oil | 10.0 % by weight |
| - water up to | 100.0 % by weight |

The lubricant, emulsifier and the emollient are melted together at 65 to 70°C. The active substance and the preservation agent are dissolved in water. The oily phase is added under constant stirring to the aqueous solution and after cooling to room temperature the desired cream is obtained.

### Example 2: Topical pharmaceutical preparation in the form of a cream with a rubefacient.

A cream of the following composition was prepared:

| | |
|---|---|
| - lysine clonixinate | 5.0 % by weight |
| - isopropyl myristate | 10.0 % by weight |
| - methyl parabene | 0.1 % by weight |
| - propyl parabene | 0.02 % by weight |
| -"emulsifier wax" | 10.0 % by weight |
| - vaseline | 10.0 % by weight |
| - menthol | 1.0 % by weight |
| - water up to | 100.0 % by weight |

The lubricant and the emulsifier were melted together at 65-70°C. The active substance and the preservation agent were dissolved in water. The oil phase is added under constant stirring to the aqueous solution.

Menthol was dissolved up to complete solution in the emollient.

After cooling to 40°C the menthol solution in the emollient was added to the emulsion with constant stirring. After cooling to room temperature the desired cream was obtained.

### Example 3: Topical pharmaceutical preparation in the form of a gel without rubefacient.

A gel of the following composition was prepared:

| | |
|---|---|
| - lysine clonixinate | 5.0 % by weight |
| - carbomer | 3.0 % by weight |
| - propylene glycol | 5.0 % by weight |
| - isopropyl myristate | 3.0 % by weight |
| - glycerol | 3.0 % by weight |
| - methyl parabene | 0.1 % by weight |
| - propyl parabene | 0.02 % by weight |
| - sodium hydroxide | 1.5 % by weight |
| - water up to | 100.0 % by weight |

The active substance was dissolved in the mixture of propylene glycol and glycerol (solution A). The preservation substances were dissolved in water, the emulsifier was dispersed in the aqueous solution and the buffer (pH-modifier) was added (dispersion B). The solution A was poured over the dispersion B and finally isopropyl myristate was added.

### Example 4: Topical pharmaceutical preparation in the form of a gel with rubefacient.

A gel of the following composition was prepared:

| | |
|---|---|
| - lysine clonixinate | 5.0 % by weight |
| - carbomer | 3.0 % by weight |
| - propylene glycol | 6.0 % by weight |
| - isopropyl myristate | 3.5 % by weight |
| - methyl parabene | 0.1 % by weight |
| - sodium hydroxide | 2.0 % by weight |
| - menthol | 1.0 % by weight |
| - water up to | 100.0 % by weight |

The active substance was dissolved in the propylene glycol and a third of the total amount of water (solution A). The preservation substance was dissolved in the remaining water and the emulsifier dispersed in said aqueous solution; thereupon, the buffer (pH-modifier) was added (dispersion B). The solution A was then poured into the dispersion B whilst stirring and finally the menthol dissolved in isopropyl myristate added. The pH-value was checked and adjusted to 8.2-8.9.

The following examples 5 and 6 will explain the advantageous use of the topical agent according to the invention compared with a conventional analgesic/anti-inflammatory agent (diclofenac) both as regards the alleviation of inflammations and the absorption or takeup (through the skin).

### Example 5

### Anti-inflammatory effect. Paw edema induced by carragheenin

Carragheenin in 1 % dosage was administered to Wistar rats of both sexes, body weight 175-200 g, that is 0.1 ml into the paw, corresponding to the procedure adopted by Winter et al. (J. Pharmacol. and Exp. Therap. 1949, 96; 99). 30 minutes previously, in each case one of the three groups of six test animals had been treated with the following compositions: lysine clonixinate cream corresponding to example 1, 5 %; 20 mg/kg; sodium diclofenac 5 %, 20 mg/kg; excipient; in each case on an area of 20 cm³ on the back of the previously shaved animals. The inflammatory edema of the paw was measured with a Ugo Basile plethysmometer 3 and 5 h after the carragheenin administration. The percentage alleviation or reduction of the inflammation was determined with respect to the control group. The results are summarized in the following Table 1.

**Table 1**

| Percentage alleviation of the inflammation by the various compounds tested | | |
|---|---|---|
| Pharmaceutical preparation | % alleviation | |
| | 3 h | 5 h |
| lysine clonixinate | 50 | 48 |
| sodium diclofenac | 35 | 30 |

### Example 6

### Transdermal absorption. Comparative tests between a cream and a gel each containing lysine clonixinate, with corresponding commercially available products

As test animals, Flemish giant rabbits having a body weight of about 3.5 kg were used which had been shaved on their backs over an area of 10 cm³.

Each group contained three animals which had been treated with the following formulations and the following doses:
a) Lysine clonixinate, 5 % cream, 20 mg/kg, prepared according to example 1.
b) Lysine clonixinate, 5 % gel, 20 mg/kg, prepared according to example 3.
c) Sodium diclofenac, 5 % cream, 20 mg/kg.
d) Diclofenac diethyl ammonium salt, 5 % gel, 20 mg/kg.

Blood samples were taken from the central artery of the ear and the concentrations of the various active substances determined.

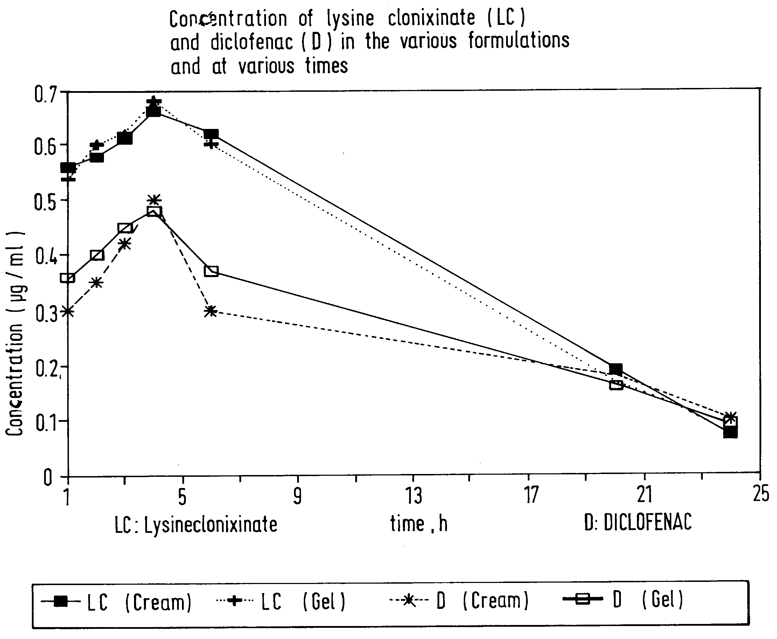

The results are shown in the following Table 2 and in the diagram attached as Fig. 1.

**Table 2**

| Concentration of lysine clonixinate and diclofenac in mg/ml in the various formulations and at various times | | | | | | | | |
|---|---|---|---|---|---|---|---|---|
| Pharmaceutical preparation | | time h | | | | | | |
| | | 1 | 2 | 3 | 4 | 6 | 20 | 24 |
| Lysine clonixinate | cream | 0,56 ± 0,11 | 0,58 ±0,12 | 0,61 ± 0,13 | 0,66 ± 0,13 | 0,62 ± 0,14 | 0,19 ± 0,04 | 0,07 ± 0,02 |
| | Gel | 0,54 ± 0,10 | 0,60 ±0,11 | 0,62 ± 0,12 | 0,68 ± 0,14 | 0,60 ± 0,12 | 0,17 ± 0,03 | 0,08 ± 0,02 |
| Diclofenac | cream | 0,30 ± 0,07 | 0,35 ±0,08 | 0,42 ± 0,08 | 0,50 ± 0,10 | 0,30 ± 0,07 | 0,18 ± 0,04 | 0,10 ± 0,02 |
| | Gel | 0,36 ± 0,06 | 0,40 ±0,08 | 0,45 ± 0,08 | 0,48 ± 0,09 | 0,37 ± 0,07 | 0,16 ± 0,03 | 0,09 ± 0,02 |

## Claims (Claims for the following Contracting State(s): AT, BE, CH, DE, DK, FR, GB, IT, LI, LU, NL, SE)

1. Pharmaceutical preparations for topical application containing 1 to 10 % by weight L-lysine salt of the 2-[(3-chloro-2-methylphenyl)amino]-3-pyridine carboxylic acid as active substance, 2 to 15 % by weight pharmaceutically acceptable emulsifiers. 2 to 20 % by weight pharmaceutically acceptable emollients, 0.01 to 0.2 % by weight pharmaceutically acceptable preservation substances and the remainder water made up to 100 % by weight, and possibly 0.2 to 5 % by weight rubefacients, the water being correspondingly adjusted to a total of 100 % by weight.

2. Pharmaceutical preparations according to claim 1,
characterized in that they are present in the form of a cream or gel and in the case of a cream additionally contain 3 to 18 % by weight pharmaceutically acceptable lubricants and in the case of a gel 0.5 to 5 % by weight pH-value modifiers (buffers), the water content being correspondingly adjusted to 100 % by weight.

3. Pharmaceutical preparations according to claim 1 or 2,
characterized in that they contain 3 to 7 % by weight of the L-lysine salt of the 2-[(3-chloro-2-methylphenyl)amino]-3-pyridine carboxylic acid as active substance, 3 to 13 % by weight pharmaceutically acceptable emulsifiers, 3 to 10 % by weight pharmaceutically acceptable emollients, 0.02 to 0.1 % by weight pharmaceutical preservation substances, and the remainder water corresponding to 100 % by weight, possibly additionally 0.5 to 2 % by weight rubefacients, and in the case of creams 8 to 12 % by weight pharmaceutically acceptable lubricants and in the case of gels 1 to 3 % by weight pH-value modifiers, the proportion of accompanying water having been correspondingly adjusted to 100 %.

4. Pharmaceutical preparations according to any one of claims 1 to 3, characterized in that the emulsifier is selected from the group consisting of glyceryl monostearate, glyceryl monostearate/polyoxyethylene stearate, ketostearyl alcohol/sodium lauryl sulfate ("emulsifier wax") and carbomer.

5. Pharmaceutical preparations according to any one of claims 1 to 4,
characterized in that the emollient is selected from the group consisting of propylene glycol, glycerol, sorbite and isopropyl myristate.

6. Pharmaceutical preparations according to any one of claims 1 to 5,
characterized in that the preservation agent is selected from the group consisting of methyl parabene, ethyl parabene and propyl parabene.

7. Pharmaceutical preparations according to any one of claims 1 to 6,
characterized in that the rubefacient is menthol.

8. Pharmaceutical preparations according to any one of claims 2 to 7,
characterized in that in the case of a cream they contain as emulsifier ketostearyl alcohol/sodium lauryl sulfate ("emulsifier wax").

9. Pharmaceutical preparations according to any one of claims 2 to 7,
characterized in that in the case of a gel they contain carbomer as emulsifier.

10. Pharmaceutical preparations according to any one of claims 2 to 7 and 9,
characterized in that they contain propylene glycol as emollient.

11. Pharmaceutical preparations according to any one of claims 2 to 10,
characterized in that they contain isopropyl myristate as emollient.

12. Pharmaceutical preparations according to any one of claims 2 to 11,
characterized in that they contain as preservation agent methyl parabene or propyl parabene.

13. Use of the L-lysine salt of the 2-[(3-chloro-2-methylphenyl)amino]-3-pyridine carboxylic acid for producing a pharmaceutical preparation for topical application which relieves/alleviates pain and/or inflammation in humans and animals.

## Claims (Claims for the following Contracting State(s): ES, GR)

1. A process for the production of pharmaceutical preparation for topical application wherein
1 to 10 % by weight L-lysine salt of the 2-[(3-chloro-2-methylphenyl)amino]-3-pyridine carboxylic acid as active substance, 2 to 15 % by weight pharmaceutically acceptable emulsifiers, 2 to 20 % by weight pharmaceutically acceptable emollients, 0.01 to 0.2 % by weight pharmaceutically acceptable preservation substances and the remainder water made up to 100 % by weight, and possibly 0.2 to 5 % by weight rubefacients, the water being correspondingly adjusted to a total of 100 % by weight are brought to a galenic form of a cream or gel.

2. Process according to claim 1, characterized in that the pharmaceutical preparations are in the form of a cream or gel and in the case of a cream additionally contain 3 to 18 % by weight pharmaceutically acceptable lubricants and in the case of a gel 0.5 to 5 % by weight pH-value modifiers (buffers), the water content being correspondingly adjusted to 100 % by weight.

3. Process according to claim 1 or 2, characterized in that the pharmaceutical preparations contain 3 to 7 % by weight of the L-lysine salt of the 2-[(3-chloro-2-methylphenyl)amino]-3-pyridine carboxylic acid as active substance, 3 to 13 % by weight pharmaceutically acceptable emulsifiers, 3 to 10 % by weight pharmaceutically acceptable emollients, 0.02 to 0.1 % by weight pharmaceutical preservation substances, and the remainder water corresponding to 100 % by weight, possibly additionally 0.5 to 2 % by weight rubefacients, and in the case of creams 8 to 12 % by weight pharmaceutically acceptable lubricants and in the case of gels 1 to 3 % by weight pH-value modifiers, the proportion of accompanying water having been correspondingly adjusted to 100 %.

4. Process according to any one of claims 1 to 3, characterized in that the emulsifier is selected from the group consisting of glyceryl monostearate, glyceryl monostearate/polyoxyethylene stearate, ketostearyl alcohol/sodium lauryl sulfate ("emulsifier wax") and carbomer.

5. Process according to any one of claims 1 to 4, characterized in that the emollient is selected from the group consisting of propylene glycol, glycerol, sorbite and isopropyl myristate.

6. Process according to any one of claims 1 to 5, characterized in that the preservation agent is selected from the group consisting of methyl parabene, ethyl parabene and propyl parabene.

7. Process according to any one of claims 1 to 6, characterized in that the rubefacient is menthol.

8. Process according to any one of claims 2 to 7, characterized in that in the case of a cream the pharmaceutical preparations contain as emulsifier ketostearyl alcohol/sodium lauryl sulfate ("emulsifier wax").

9. Process according to any one of claims 2 to 7, characterized in that in the case of a gel the pharmaceutical preparations contain carbomer as emulsifier.

10. Process according to any one of claims 2 to 7 and 9, characterized in that the pharmaceutical preparations contain propylene glycol as emollient.

11. Process according to any one of claims 2 to 10, characterized in that the pharmaceutical preparations contain isopropyl myristate as emollient.

12. Process according to any one of claims 2 to 11, characterized in that the pharmaceutical preparations contain as preservation agent methyl parabene or propyl parabene.

13. Use of the L-lysine salt of the 2-[(3-chloro-2-methylphenyl)amino]-3-pyridine carboxylic acid for producing a pharmaceutical preparation for topical application which relieves/alleviates pain and/or inflammation in humans and animals.

14. A process for the production of a topical pharmaceutical preparation containing lysine clonixinate as active substance and in the form of a cream without rubefacient wherein lubricant, emulsifier and emollient are melted together at 65 to 75°C and added to an aqueous solution of the active substance and a preservation agent under stirring.

15. A process for the production of a topical pharmaceutical preparation containing lysine clonixinate as active substance and in the form of a cream with a rubefacient wherein a lubricant and an emulsifier are melted together at 65 to 70°C, and added to an aqueous solution of the active substance and a preservation agent under stirring; followed by the addition of a solution of the rubefacient in an emollient after cooling to 40°C under stirring.

16. A process for the production of a topical pharmaceutical preparation containing lysine clonixinate as active substance and in the form of a gel without rubefacient wherein a solution A, prepared by dissolving the active substance in a mixture of propylene glycol and glycerol is poured over a dispersion B, prepared by dissolving a preservation agent in water and dispersing an emulsifier therein; and finally isopropyl myristate is added.

17. A process for the production of a topical pharmaceutical preparation containing lysine clonixinate as active substance and in the form of gel with rubefacient wherein the active substance is dissolved in propylene glycol and a third of the total amount of water (solution A), and a preservation substance is dissolved in the remaining water and an emulsifier dispersed therein forming a dispersion B, followed by pouring solution A into dispersion B under stirring and adding a solution of the rubefacient in an emollient.

## Patentansprüche (Patentansprüche für folgende(n) Vertragsstaat(en): AT, BE, CH, DE, DK, FR, GB, IT, LI, LU, NL, SE)

1. Arzneimittel zur topischen Anwendung enhaltend 1 bis 10 Gew.-% L-Lysinsalz der 2-[(3-Chlor-2-methylphenyl)amino]-3-pyridincarbonsäure als Wirkstoff, 2 bis 15 Gew.-% pharmazeutisch annehmbare Emulgatoren, 2 bis 20 Gew.-% pharmazeutisch annehmbare Emollienten, 0,01 bis 0,2 Gew.-% pharmazeutisch annehmbare Konservierungsstoffe und Rest Wasser auf 100 Gew.-% sowie ggf. 0,2 bis 5 Gew.-% Rubefazientien, wobei das Wasser entsprechend auf insgesamt 100 Gew.-% eingestellt ist.

2. Arzneimittel nach Anspruch 1,
dadurch **gekennzeichnet**, daß sie in Form einer Creme oder eines Gels vorliegen und im Falle einer Creme zusätzlich 3 bis 18 Gew.-% pharmazeutisch annehmbare Gleitmittel sowie im Falle eines Gels 0,5 bis 5 Gew.-% pH-Wert Modifikatoren (Puffer) enthalten, wobei der Wasseranteil entsprechend 100 Gew.-% eingestellt ist.

3. Arzneimittel nach Anspruch 1 oder 2,
dadurch **gekennzeichnet**, daß sie 3 bis 7 Gew.-% L-Lysinsalz der 2-[(3-Chlor-2-methylphenyl)amino]3-pyridincarbonsäure als Wirkstoff, 3 bis 13 Gew.-% pharmazeutisch annehmbare Emulgatoren, 3 bis 10 Gew.-% pharmazeutisch annehmbare Emollienten, 0,02 bis 0,1 Gew.-% pharmazeutische Konservierungsstoffe, Rest Wasser entsprechend 100 Gew.-% enthalten, ggf. zusätzlich 0,5 bis 2 Gew.-% Rubefazientien, sowie im Falle von Cremes 8 bis 12 Gew.-% pharmazeutisch annehmbare Gleitmittel und im Falle von Gelen 1 bis 3 Gew.-% pH-Wert Modifikatoren, wobei der Anteil an begleitendem Wasser entprechend 100 % eingestellt worden ist.

4. Arzneimittel nach einem der Ansprüche 1 bis 3,
dadurch **gekennzeichnet**, daß der Emulgator aus der Gruppe bestehend aus Glycerylmonostearat, Glycerylmonostearat/Polyoxyethylenstearat, Ketostearylalkohol/Natriumlaurylsulfat ("Emulgierwachs") und Carbomer ausgewählt ist.

5. Arzneimittel nach einem der Ansprüche 1 bis 4,
dadurch **gekennzeichnet**, daß das Emolliens aus der Gruppe bestehend aus Propylenglykol, Glycerin, Sorbit und Isopropylmyristat ausgewählt ist.

6. Arzneimittel nach einem der Ansprüche 1 bis 5;
dadurch **gekennzeichnet**, daß das Konservierungsmittel aus der Gruppe bestehend aus Methylparaben, Ethylparaben und Propylparaben ausgewählt ist.

7. Arzneimittel nach einem der Ansprüche 1 bis 6 ,
dadurch **gekennzeichnet**, daß der Rubefazient Menthol ist.

8. Arzneimittel nach einem der Ansprüche 2 bis 7,
dadurch **gekennzeichnet**, daß sie im Falle einer Creme als Emulgator Ketostearylalkohol/Natriumlaurylsulfat ("Emulgierwachs") enthalten.

9. Arzneimittel nach einem der Ansprüche 2 bis 7,
dadurch **gekennzeichnet**, daß sie im Falle eines Gels als Emulgator Carbomer enthalten.

10. Arzneimittel nach einem der Ansprüche 2 bis 7 und 9 ,
dadurch **gekennzeichnet**, daß sie als Emolliens Propylenglykol enthalten,

11. Arzneimittel, nach einem der Ansprüche 2 bis 10,
dadurch **gekennzeichnet**, daß sie als Emolliens Isopropylmyristat enthalten.

12. Arzneimittel nach einem der Ansprüche 2 bis 11,
dadurch **gekennzeichnet**, daß sie als Konservierungsmittel Methylparaben oder Propylparaben enthalten.

13. Verwendung des L-Lysinsalzes der [(3-Chlor-2-methylphenyl)amino]-3-pyridincarbonsäure zur Herstellung eines Arzneimittels zur topischen Anwendung, das Schmerz und/oder Entzündung bei Mensch oder Tier behebt/lindert.

## Patentansprüche (Patentansprüche für folgende(n) Vertragsstaat(en): ES, GR)

1. Verfahren zur Herstellung einer pharmazeutischen Präparation zur topischen Anwendung, wobei 1 bis 10 Gew.-% L-Lysinsalz der 2-[(3-Chlor-2-methylphenyl)amino]-3-pyridincarbonsäure als Wirkstoff, 2 bis 15 Gew.-% pharmazeutisch annehmbare Emulgatoren, 2 bis 20 Gew.-% pharmazeutisch annehmbare Emollienten, 0,01 bis 0,2 Gew.-% pharmazeutisch annehmbare Konservierungsstoffe und Rest Wasser auf 100 Gew.-% sowie gegebenenfalls 0,2 bis 5 Gew.-% Rubefazientien, wobei das Wasser entsprechend auf insgesamt 100 Gew.-% eingestellt ist, in die galenische Form einer Creme oder eines Gels gebracht werden.

2. Verfahren nach Anspruch 1, dadurch gekennzeichnet, daß die pharmazeutische Präparation in Form einer Creme oder eines Gels vorliegt und im Falle einer Creme zusätzlich 3 bis 18 Gew.-% pharmazeutisch annehmbare Gleitmittel sowie im Falle eines Gels 0,5 bis 5 Gew.-% pH-Wert Modifikatoren (Puffer) enthalten, wobei der Wasseranteil auf 100 Gew.-% eingestellt ist.

3. Verfahren nach Anspruch 1 oder 2, dadurch gekennzeichnet, daß die pharmazeutische Präparation 3 bis 7 Gew.-% L-Lysinsalz der 2-[(3-Chlor-2-methylphenyl)amino]-3-pyridincarbonsäure als Wirkstoff, 3 bis 13 Gew.-% pharmazeutisch annehmbare Emulgatoren, 3 - 10 Gew.-% pharmazeutisch annehmbare Emollienten, 0,02 bis 0,1 Gew.-% pharmazeutische Konservierungsstoffe, Rest Wasser entsprechend 100 Gew.-% enthalten, gegebenenfalls zusätzlich 0,5 bis 2 Gew.-% Rubefazientien, sowie im Falle von Cremes 8 bis 12 Gew.-% pharmazeutisch annehmbare Gleitmittel und im Falle von Gelen 1 bis 3 Gew.-% pH-Wert Modifikatoren, wobei der Anteil an begleitendem Wasser entsprechend 100 % eingestellt ist.

4. Verfahren nach einem der Ansprüche 1 bis 3, dadurch gekennzeichnet, daß der Emulgator aus der Gruppe bestehend aus Glycerylmonostearat, Glycerylmonostearat/Polyoxyethylenstearat, Ketostearylalkohol/Natriumlaurylsulfat ("Emulgierwachs") und Carbomer ausgewählt ist.

5. Verfahren nach einem der Ansprüche 1 bis 4, dadurch gekennzeichnet, daß das Emolliens aus der Gruppe bestehend aus Propylenglykol, Glycerin, Sorbit und Isopropylmyristat ausgewählt ist.

6. Verfahren nach einem der Ansprüche 1 bis 5, dadurch gekennzeichnet, daß das Konservierungsmittel aus der Gruppe bestehend aus Methylparaben, Ethylparaben und Propylparaben ausgewählt ist.

7. Verfahren nach einem der Ansprüche 1 bis 6, dadurch gekennzeichnet, daß der Rubefazient Menthol ist.

8. Verfahren nach einem der Ansprüche 2 bis 7, dadurch gekennzeichnet, daß im Falle einer Creme die pharmazeutische Präparation als Emulgator Ketostearylalkohol/Natriumlaurylsulfat ("Emulgierwachs") enthält.

9. Verfahren nach einem der Ansprüche 2 bis 7, dadurch gekennzeichnet, daß im Falle eines Gels die pharmazeutische Präparation Carbomer als Emulgator enthält.

10. Verfahren nach einem der Ansprüche 2 bis 7 und 9, dadurch gekennzeichnet, daß die pharmazeutische Präparation Propylenglykol als Emolliens enthält.

11. Verfahren nach einem der Ansprüche 2 bis 10, dadurch gekennzeichnet, daß die pharmazeutische Präparation Isopropylmyristat als Emolliens enthält.

12. Verfahren nach einem der Ansprüche 2 bis 11, dadurch gekennzeichnet, daß die pharmazeutische Präparation als Konservierungsmittel Methylparaben oder Propylparaben enthält.

13. Verwendung des L-Lysinsalzes der [3(-Chlor-2-methylphenyl)amino]-3-pyridincarbonsäure zur Herstellung eines Arzneimittels zur topischen Anwendung, das Schmerz und/oder Entzündung bei Mensch oder Tier behebt/lindert.

14. Verfahren zur Herstellung einer topischen pharmazeutischen Präparation enthaltend Lysinclonixinat als aktive Substanz und in der Form einer Creme ohne Rubefazient, wobei Gleitmittel, Emulgator und Emolliens bei 65 bis 75°C zusammengeschmolzen werden und einer wäßrigen Lösung der aktiven Substanz und eines Konservierungsmittels unter Rühren zugegeben werden.

15. Verfahren zur Herstellung einer topischen pharmazeutischen Präparation enthaltend Lysinclonixinat als aktive Substanz und in Form einer Creme mit einem Rubefazient, wobei ein Gleitmittel und ein Emulgator bei 65 bis 70°C zusammengeschmolzen werden und einer wäßrigen Lösung der aktiven Substanz und eines Konservierungsmittels unter Rühren zugegeben wird, gefolgt durch Zugabe einer Lösung des Rubefazienten in einem Emolliens nach Kühlen auf 40°C unter Rühren.

16. Verfahren zur Herstellung einer topischen pharmazeutischen Präparation enthaltend Lysinclonixinat als aktive Substanz und in der Form eines Gels ohne Rubefazient, wobei eine Lösung A, hergestellt durch Auflösen der aktiven Substanz in einer Mischung aus Propylenglykol und Glycerol über eine Dispersion B, die durch Auflösung eines Konservierungsmittels in Wasser und Dispergierung eines Emulgators darin hergestellt wurde, gegossen wird; und schließlich wird Isopropylmyristat zugegeben.

17. Verfahren zur Herstellung einer topischen pharmazeutischen Präparation enthaltend Lysinclonixinat als aktive Substanz und in der Form eines Gels mit Rubefazient, wobei die aktive Substanz in Propylenglykol und einem Drittel der Gesamtwassermenge aufgelöst wird (Lösung A), und es wird eine konservierende Substanz in dem Rest Wasser aufgelöst und unter Bildung einer Dispersion B ein Emulgator darin dispergiert, gefolgt von einem Gießen der Lösung A in die Dispersion B unter Rühren und Zugabe einer Lösung des Rubefazienten in einen Emolliens.

## Revendications (Revendications pour l'(les) Etat(s) contractant(s) suivant(s): AT, BE, CH, DE, DK, FR, GB, IT, LI, LU, NL, SE)

1. Préparations pharmaceutiques destinées à être administrées par voie topique contenant 1 à 10 % en masse de sel de L-lysine de l'acide 2-[(3-chloro-2-méthylphényl)amino]-3-pyridinecarboxylique en tant que substance active, 2 à 15 % en masse d'émulsifiants pharmaceutiquement acceptables, 2 à 20 % en masse d'émollients pharmaceutiquement acceptables, 0,01 à 0,2 % en masse de substances conservatrices pharmaceutiquement acceptables, le tout complété à 100 % en masse par de l'eau, et contenant éventuellement 0,2 à 5 % en masse de rubéfiants, la quantité d'eau étant ajustée proportionnellement pour faire un total de 100 % en masse.

2. Préparations pharmaceutiques selon la revendication 1, caractérisées en ce qu'elles sont sous forme de crème ou de gel et que, dans le cas de crèmes, elles contiennent en outre 3 à 18 % en masse de lubrifiants pharmaceutiquement acceptables et, dans le cas de gels, elles contiennent 0,5 à 5 % en masse de modificateurs du pH (tampons), la quantité d'eau étant ajustée proportionnellement pour faire un total de 100 % en masse.

3. Préparations pharmaceutiques selon la revendication 1 ou 2, caractérisées en ce qu'elles contiennent 3 à 7 % en masse de sel de L-lysine de l'acide 2-[(3-chloro-2-méthylphényl)amino]-3-pyridinecarboxylique en tant que substance active, 3 à 13 % en masse d'émulsifiants pharmaceutiquement acceptables, 3 à 10 % en masse d'émollients pharmaceutiquement acceptables, 0,02 à 0,1 % en masse de substances conservatrices pharmaceutiquement acceptables, le tout complété à 100 % en masse par de l'eau, et en ce qu'elles contiennent éventuellement 0,5 à 2 % en masse de rubéfiants et, dans le cas de crèmes, 8 à 12 % en masse de lubrifiants pharmaceutiquement acceptables et, dans le cas de gels, 1 à 3 % en masse de modificateurs du pH, la quantité d'eau étant ajustée proportionnellement pour faire un total de 100 % en masse.

4. Préparations pharmaceutiques selon une quelconque des revendications 1 à 3, caractérisées en ce que l'émulsifiant est choisi dans le groupe comprenant le monostéarate de glycéryle, le monostéarate de glycéryle/stéarate de polyoxyéthylèneglycol, l'alcool cétostéarylique/laurylsulfate de sodium (cire émulsifiante) et le carbomère.

5. Préparations pharmaceutiques selon une quelconque des revendications 1 à 4, caractérisées en ce que l'émollient est choisi dans le groupe constitué par le propylèneglycol, le glycérol, le sorbitol et le myristate d'isopropyle.

6. Préparations pharmaceutiques selon une quelconque des revendications 1 à 5, caractérisées en ce que l'agent conservateur est choisi dans le groupe constitué par le p-hydroxybenzoate de méthyle, le p-hydroxybenzoate d'éthyle et le p-hydroxybenzoate de propyle.

7. Préparations pharmaceutiques selon une quelconque des revendications 1 à 6, caractérisées en ce que le rubéfiant est le menthol.

8. Préparations pharmaceutiques selon une quelconque des revendications 2 à 7, caractérisées en ce que, dans le cas où il s'agit de crèmes, elles contiennent comme émulsifiant l'alcool cétostéarylique/laurylsulfate de sodium ("cire émulsifiante").

9. Préparations pharmaceutiques selon une quelconque des revendications 2 à 7, caractérisées en ce que, dans le cas où il s'agit de gels, elles contiennent du carbomère comme émulsifiant.

10. Préparations pharmaceutiques selon une quelconque des revendications 2 à 7 et 9, caractérisées en ce qu'elles contiennent du propylèneglycol en tant qu'émollient.

11. Préparations pharmaceutiques selon une quelconque des revendications 2 à 10, caractérisées en ce qu'elles contiennent du myristate d'isopropyle en tant qu'émollient.

12. Préparations pharmaceutiques selon une quelconque des revendications 2 à 11, caractérisées en ce qu'elles contiennent en tant qu'agent de conservation du p-hydroxybenzoate de méthyle ou du p-hydroxybenzoate de propyle.

13. Utilisation du sel de L-lysine de l'acide 2-[(3-chloro-2-méthylphényl)amino]-3-pyridinecarboxylique pour la production d'une préparation pharmaceutique destinée à une administration par voie topique afin de guérir ou soulager la douleur et/ou les inflammations chez l'homme et l'animal.

## Revendications (Revendications pour l'(les) Etat(s) contractant(s) suivant(s): ES, GR)

1. Procédé pour la production d'une préparation pharmaceutique destinée à être administrée par voie topique par lequel 1 à 10 % en masse de sel de L-lysine de l'acide 2-[(3-chloro-2-méthylphényl)amino]-3-pyridinecarboxylique en tant que substance active, 2 à 15 % en masse d'émulsifiants pharmaceutiquement acceptables, 2 à 20 % en masse d'émollients pharmaceutiquement acceptables, 0,01 à 0,2 % en masse de substances conservatrices pharmaceutiquement acceptables, le tout complété à 100 % en masse par de l'eau, avec éventuellement 0,2 à 5 % en masse de rubéfiants, la quantité d'eau étant ajustée proportionnellement pour faire un total de 100 % en masse, sont transformés en une forme galénique constituant une crème ou un gel.

2. Procédé selon la revendication 1, caractérisé en ce que les préparations pharmaceutiques sont sous forme de crème ou de gel et en ce que, dans le cas de crèmes, elles contiennent en outre 3 à 18 % en masse de lubrifiants pharmaceutiquement acceptables et, dans le cas de gels, elles contiennent 0,5 à 5 % en masse de modificateurs du pH (tampons), la quantité d'eau étant ajustée proportionnellement pour faire un total de 100 % en masse.

3. Procédé selon la revendication 1 ou 2, caractérisé en ce que les préparations pharmaceutiques contiennent 3 à 7 % en masse de sel de L-lysine de l'acide 2-[(3-chloro-2-méthylphényl)amino]-3-pyridinecarboxylique en tant que substance active, 3 à 13 % en masse d'émulsifiants pharmaceutiquement acceptables, 3 à 10 % en masse d'émollients pharmaceutiquement acceptables, 0,02 à 0,1 % en masse de substances conservatrices pharmaceutiquement acceptables, le tout complété à 100 % en masse par de l'eau, avec éventuellement 0,5 à 2 % en masse de rubéfiants et avec, dans le cas de crèmes, 8 à 12 % en masse de lubrifiants pharmaceutiquement acceptables et, dans le cas de gels, 1 à 3 % en masse de modificateurs du pH, la quantité d'eau étant ajustée proportionnellement pour faire un total de 100 % en masse.

4. Procédé selon une quelconque des revendications 1 à 3, caractérisé en ce que l'émulsifiant est choisi dans le groupe comprenant le monostéarate de glycéryle, le monostéarate de glycéryle/stéarate de polyoxyéthylèneglycol, l'alcool cétostéarylique/ laurylsulfate de sodium (cire émulsifiante) et le carbomère.

5. Procédé selon une quelconque des revendications 1 à 4, caractérisé en ce que l'émollient est choisi dans le groupe constitué par le propylèneglycol, le glycérol, le sorbitol et le myristate d'isopropyle.

6. Procédé selon une quelconque des revendications 1 à 5, caractérisé en ce que l'agent conservateur est choisi dans le groupe constitué par le p-hydroxybenzoate de méthyle, le p-hydroxybenzoate d'éthyle et le p-hydroxybenzoate de propyle.

7. Procédé selon une quelconque des revendications 1 à 6, caractérisé en ce que le rubéfiant est le menthol.

8. Procédé selon une quelconque des revendications 2 à 7, caractérisé en ce que, dans le cas où il s'agit de crèmes, les préparations pharmaceutiques contiennent comme émulsifiant l'alcool cétostéarylique/laurylsulfate de sodium ("cire émulsifiante").

9. Procédé selon une quelconque des revendications 2 à 7, caractérisé en ce que, dans le cas où il s'agit de gels, les préparations pharmaceutiques contiennent du carbomère comme émulsifiant.

10. Procédé selon une quelconque des revendications 2 à 7 et 9, caractérisé en ce que les préparations pharmaceutiques contiennent du propylèneglycol en tant qu'émollient.

11. Procédé selon une quelconque des revendications 2 à 10, caractérisé en ce que les préparations pharmaceutiques contiennent du myristate d'isopropyle en tant qu'émollient.

12. Procédé selon une quelconque des revendications 2 à 11, caractérisé en ce que les préparations pharmaceutiques contiennent en tant qu'agent conservateur du p-hydroxybenzoate de méthyle ou du p-hydroxybenzoate de propyle.

13. Utilisation du sel de L-lysine de l'acide 2-[(3-chloro-2-méthylphényl)amino]-3-pyridinecarboxylique pour la production d'une préparation pharmaceutique destinée à une administration par voie topique afin de guérir ou soulager la douleur et/ou les inflammations chez l'homme et l'animal.

14. Procédé de production d'une préparation pharmaceutique topique contenant le clonixinate de lysine en tant que substance active et sous la forme d'une crème ne contenant pas de rubéfiant, dans lequel un lubrifiant, un émulsifiant et un émollient sont fondus ensemble entre 65 et 75°C et ajoutés à une solution aqueuse de la substance active et d'un agent conservateur, sous agitation.

15. Procédé de production d'une préparation pharmaceutique topique contenant le clonixinate de lysine en tant que substance active et sous la forme d'une crème contenant un rubéfiant, dans lequel un lubrifiant et un émulsifiant sont fondus ensemble entre 65 et 70°C et ajoutés à une solution aqueuse de la substance active et d'un agent conservateur, sous agitation, opération suivie de l'addition d'une solution du rubéfiant dans un émollient après refroidissement jusqu'à 40°C sous agitation.

16. Procédé pour la production d'une préparation pharmaceutique topique contenant le clonixinate de lysine en tant que substance active et sous la forme d'un gel ne contenant pas de rubéfiant, dans lequel une solution A, préparée en dissolvant la substance active dans un mélange de propylèneglycol et de glycérol, est versée dans une dispersion B, préparée en dissolvant un agent conservateur dans de l'eau et en y dispersant un émulsifiant, et enfin le myristate d'isopropyle est ajouté.

17. Procédé pour la production d'une préparation pharmaceutique topique contenant le clonixinate de lysine comme substance active et sous la forme d'un gel contenant un rubéfiant, dans lequel la substance active est dissoute dans du propylèneglycol et un tiers de la quantité totale d'eau (solution A) et une substance conservatrice est dissoute dans l'eau qui reste et un émulsifiant y est dispersé en formant une dispersion B, opération suivie de celle consistant à verser la solution A dans la dispersion B tout en agitant et de l'addition d'une solution du rubéfiant dans un émollient.
